# EUROPEAN PATENT SPECIFICATION

(11) **EP 3 626 872 B1**
(45) Date of publication and mention of the grant of the patent: **11.06.2025**
(21) Application number: 18796568.6
(22) Date of filing: 22.03.2018
(51) Int. Cl.: D02G 3/02

(54) **ANTIBACTERIAL YARN, SEAT, AND SEAT COVER**
ANTIBAKTERIELLES GARN, SITZ UND SITZBEZUG
FIL ANTIBACTÉRIEN, SIÈGE ET HOUSSE DE SIÈGE

(30) Priority: 19.05.2017 JP 2017099508
(43) Date of publication of application: 25.03.2020
(73) Proprietor: Murata Manufacturing Co., Ltd., Nagaokakyo-shi, Kyoto 617-8555 (JP)
(72) Inventor: ANDO, Masamichi, Nagaokakyo-shi Kyoto 617-8555 (JP)
(74) Representative: Reddie & Grose LLP
(86) International application number: PCT/JP2018/011317
(87) International publication number: WO 2018/211817

(56) References cited:
- WO-A1-2014/161920
- WO-A1-2015/159832
- WO-A1-2016/147713
- WO-A1-2016/188853
- WO-A1-2017/212523
- JP-A- 2015 198 154
- US-A1- 2010 227 521

## Description

### Technical Field

The present invention relates to an antibacterial twisted yarn that has an antimicrobial property.

### Background Art

Conventionally, many proposals have been made on fiber materials that have antimicrobial properties (see Patent Documents 1 to 6).
Patent Document 1: Japanese Patent No. 3281640
Patent Document 2: Japanese Patent Application Laid-Open No. 7-310284
Patent Document 3: Japanese Patent No. 3165992
Patent Document 4: Japanese Patent Application Laid-Open No. 8-226078
Patent Document 5: Japanese Patent Application Laid-Open No. 9-194304
Patent Document 6: Japanese Patent Application Laid-Open No. 2004-300650

However, the materials that have antimicrobial properties have all failed to last long for effect.

In addition, the materials that have antimicrobial properties may cause allergic reactions with a drug or the like.

Therefore, we have appreciated that it would be desirable to provide an antibacterial yarn which lasts longer for effect than conventional materials with antibacterial properties, and which is safer than drugs and the like.

JP-A-2015-198154 discloses a piezoelectric element including a piezoelectric unit that includes two conductive fibers and one piezoelectric fiber, the fibers having no points of mutual contact to each other, and being arranged generally on the same plane in the order of the conductive fiber, the piezoelectric fiber and the conductive fiber; and having a distance between the surfaces of the fibers in the range of 4 mm or less. The piezoelectric fiber has piezoelectric properties, and is made of a piezoelectric polymer such as polylactic acid.

US-A-2010/227521 discloses a woven active fiber composite. The woven active fiber composite includes actuating fibers interwoven with conductive wire electrodes. A method of making the woven active fiber composite is also disclosed.

WO-A-2014/161920 discloses a method for producing a piezoelectric and pyroelectric fiber comprising: providing a fiber including a core having a core material and a surrounding material enclosing the core material. The core material comprises an electrically conductive flexible thermoplastic composite comprising at least one polymer and at least one conductive filler, and the surrounding material is a permanently polarizable polymer. The method includes stretching the fiber along an elongation direction of the fiber with a draw ratio sufficient to introduce beta-crystallites in the fiber; electrically grounding the core material; while the core material is grounded, passing the fiber through an annular poling element; and applying a voltage between the annular poling element and the core material sufficient to establish an electric field for corona poling, whereby the core material is exposed to the electric field electrically charging the surrounding material.

### SUMMARY OF THE INVENTION

The invention is defined by the independent claim to which reference should now be made. Advantageous features are set forth in the dependent claims.

Conventionally, it has been known that the proliferation of bacteria, fungi, and the like can be inhibited by an electric field (see, for example, Tetsuaki Doito, Hiroshi Koryo, Hideaki Matsuoka, Junichi Koizumi, Kodansha: Microbial Control-Science and Engineering, see for example, Koichi Takagi, Application of High Voltage Plasma Technology to Agriculture Food Field, and see J. HTSJ, Vol. 51, No. 216). In addition, due to the electric potential that generates the electric field, electric current may flow through a current pathway formed by moisture or the like, or a circuit formed by a local micro discharge phenomenon or the like. This electric current is considered to weaken bacteria and inhibit the proliferation of bacteria. The antibacterial yarn according to the present invention includes a plurality of charge generation fibers that generate electric charges with external energy, and thus generates an electric field when the antibacterial yarn is brought between fibers, or close to an object with a predetermined electric potential (including the ground potential), such as a human body. Alternatively, the antibacterial yarn of the present invention allows current to flow when moisture such as perspiration is brought close to an object having a predetermined potential (including a ground potential) such as a human body or the like, between fibers and fibers.

Therefore, the antibacterial yarn according to the present invention exerts an antibacterial effect for the following reasons. The direct action of an electric field or an electric current that is generated when the antibacterial yarn is applied to an object (clothing, footwear, or a medical supply such as a mask) for use close to an object with a predetermined potential, such as a human body, poses a problem for cell membranes of bacteria or an electron transfer system for maintaining the lives of bacteria, thereby killing the bacteria, or weakening the bacteria themselves. Furthermore, oxygen included in water may be changed to active oxygen species by an electric field or an electric current, or oxygen radicals may be generated in cells of bacterium due to the stress environment in the presence of an electric field or an electric current, and the action of the active oxygen species including radicals kills or weakens bacteria. In addition, the above-mentioned reasons may be combined to produce antibacterial effect in some cases. It is to be noted that the term "antimicrobial" in the present invention refers to a concept that includes both an effect of suppressing the generation of bacteria and an effect of killing bacteria.

It is to be noted that the charge generation fiber that generates charges with external energy comprises a piezoelectric polymer.

When a piezoelectric body is used, an electric field is generated in a piezoelectric manner, and thus, no power supply is required, and there is no risk of electric shock. In addition, the lifetime of the piezoelectric body lasts longer than the antibacterial effect of a drug or the like. In addition, the piezoelectric body is less likely to cause an allergic reaction than drugs.

According to the present invention, an antibacterial yarn can be achieved which lasts longer for effect than conventional materials with antibacterial properties, and which is safer than drugs and the like.

### BRIEF EXPLANATION OF DRAWINGS

FIG. 1(A) is the configuration of an antibacterial yarn 1,
FIG. 1(B) is a cross-sectional view taken along the line A-A in FIG. 1(A), and FIG. 1(C) is a cross-sectional view taken along the line B-B of FIG. 1(A).
FIGS. 2(A) and 2(B) are diagrams showing the relationship among the direction of uniaxially stretching polylactic acid, an electric field direction, and the deformation of a piezoelectric fiber 10.
FIG. 3(A) is the configuration of an antibacterial yarn 2, FIG. 3(B) is a cross-sectional view taken along the line A-A in FIG. 3(A), and FIG. 3(C) is a cross-sectional view taken along the line B-B of FIG. 3(A).
FIG. 4(A) is a diagram showing electric potentials in the antibacterial yarn 1 and the antibacterial yarn 2, and FIG. 4(B) is a diagram showing, as a comparative example, electric potentials in the case where the spaces between the plurality of piezoelectric fibers 10 are uniform in the antibacterial yarn 1 and the antibacterial yarn 2 (comparative example).
FIG. 5(A) is a diagram showing an electric field, and FIG. 5(B) is a diagram showing, as a comparative example, an electric field in the case where the spaces between the plurality of piezoelectric fibers 10 are uniform in the antibacterial yarn 1 and the antibacterial yarn 2 (comparative example).
FIG. 6(A) is a partially exploded view illustrating the configuration of an antibacterial yarn 2A according to Modification Example 1, FIG. 6(B) is a cross-sectional view along the line A-A in FIG. 6(A), and FIG. 6(C) is a cross-sectional view taken along the line B-B of FIG. 6(A).
FIG. 7(A) is a partially exploded view illustrating the configuration of an antibacterial yarn 2B according to Modification Example 2, FIG. 7(B) is a cross-sectional view along the line A-A in FIG. 7 (A), and FIG. 7(C) is a cross-sectional view taken along the line B-B in FIG. 7(A).

### DETAILED DESCRIPTION

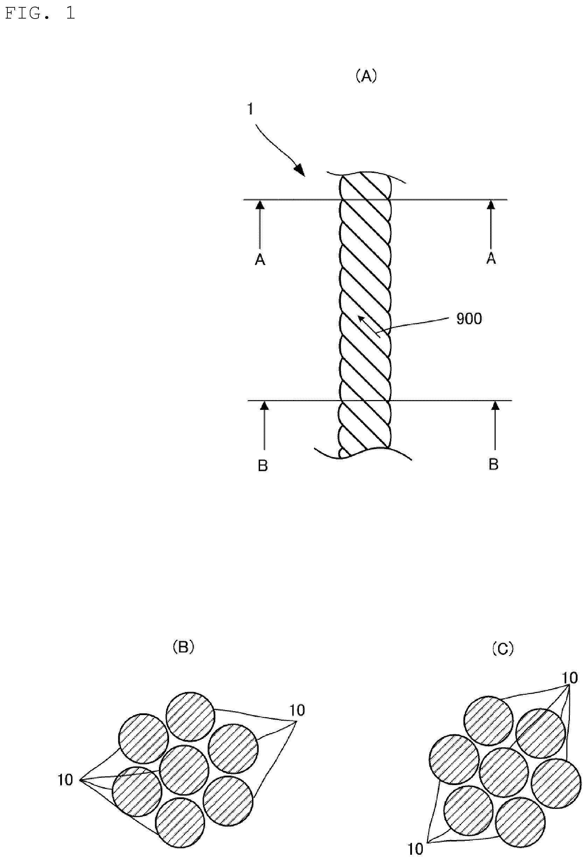

FIG. 1(A) is a partially exploded view illustrating the constitution of an antibacterial yarn 1 obtained by twisting together piezoelectric fibers 10, and FIG. 1 (B) is a cross sectional view taken along the line A-A of FIG. 1 (A). FIG. 1(C) is a cross-sectional view taken along the line B-B of FIG. 1(A).

The piezoelectric fiber 10 is a charge generation fiber that generates electric charges with external energy.

The piezoelectric fiber 10 is made of a piezoelectric polymer. Some of piezoelectric polymers have pyroelectricity, and the others have no pyroelectricity. For example, PVDF (polyvinylidene fluoride) with pyroelectricity has electric charges generated also with changes in temperature. The piezoelectric body with pyroelectricity, such as PVDF, has, at the surface thereof, electric charges generated also with thermal energy of a human body.

In addition, polylactic acid (PLA) is a piezoelectric polymer that has no pyroelectricity. Polylactic acid is uniaxially stretched to produce piezoelectricity. Polylactic acid includes PLLA in which an L-monomer is polymerized and PDLA in which a D-monomer is polymerized.

Polylactic acid is a chiral polymer, where a main chain has a helical structure. Polylactic acid develops piezoelectricity when the polylactic acid is uniaxially stretched to orient molecules. When the degree of crystallization is further increased by further applying a heat treatment, the piezoelectric constant is increased. The piezoelectric fiber 10 made of uniaxially stretched polylactic acid has tensor components of d₁₄ and d₂₅ as piezoelectric strain constants, when the thickness direction is defined as a first axis, the stretching direction 900 is defined as a third axis, and the direction orthogonal to both the first axis and the third axis is defined as a second axis. Therefore, polylactic acid generates electric charges most efficiently when strain is produced in the direction at 45 degrees with respect to the uniaxially stretched direction.

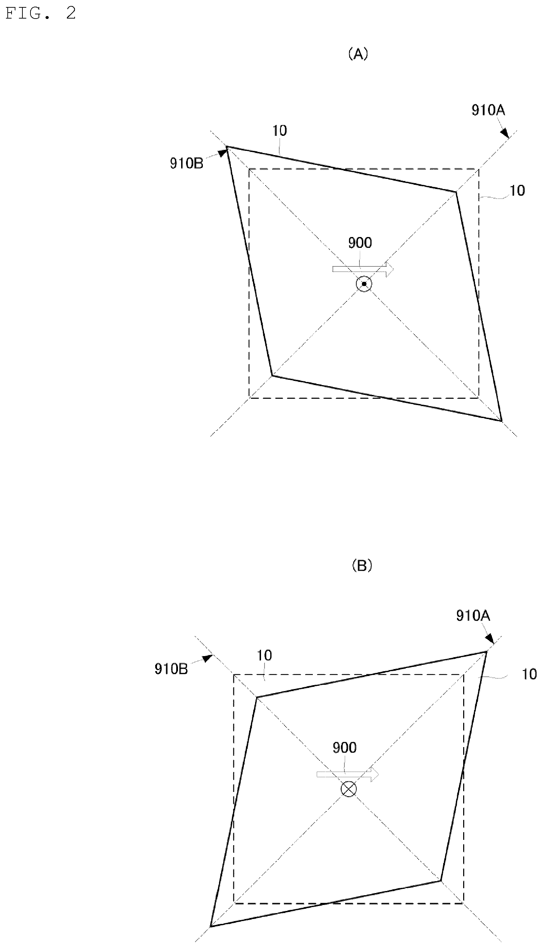

FIGS. 2(A) and 2(B) are diagrams showing the relationship among the direction of uniaxially stretching polylactic acid, an electric field direction, and the deformation of a piezoelectric fiber 10. As shown in FIG. 2(A), when the piezoelectric fiber 10 contracts in the direction of a first diagonal line 910A and extends in the direction of a second diagonal line 910B orthogonal to the first diagonal line 910A, the piezoelectric fiber 10 generates an electric field in a direction from the lower side of the paper surface toward the upper side thereof. More specifically, the piezoelectric fiber 10 has a negative electric charge generated on the upper side of the paper surface. Even when the piezoelectric fiber 10 extends in the direction of the first diagonal line 910A and contracts in the direction of the second diagonal line 910B as shown in FIG. 2(B), an electric charge is generated, but the polarity is reversed, and an electric field is generated in a direction from the upper surface of the paper surface toward the lower surface thereof. More specifically, the piezoelectric fiber 10 has a positive electric charge generated on the upper side of the paper surface.

Since polylactic acid produces piezoelectricity by a treatment of orientating molecules by stretching, there is no need to perform a poling treatment unlike other piezoelectric polymers such as PVDF or piezoelectric ceramics. The piezoelectric constant of the uniaxially stretched polylactic acid is about 5 to 30 pC/N, and the polylactic acid has a very high piezoelectric constant among polymers. Furthermore, the piezoelectric constant of polylactic acid is extremely stable without varying with time.

The piezoelectric fiber 10 is a fiber that is circular in cross section. The piezoelectric fiber 10 can be produced by, for example, an approach of extruding a piezoelectric polymer for fiber formation, an approach of melt-spinning a piezoelectric polymer for fiber formation (including, for example, a spinning-drawing method of separately performing a spinning step and a drawing step, a direct drawing method of connection a spinning step and a drawing step, a POY-DTY method capable of also performing a temporary twisting step at the same time, an ultrahigh-speed spinning method for speeding up, or the like), an approach of forming a piezoelectric polymer into a fiber by dry or wet spinning (including, for example, a phase separation method or a dry-wet spinning method of dissolving a polymer as a raw material in a solvent and extruding the dissolved polymer from a nozzle for fiber formation, a gel spinning method of uniformly forming a fiber in the form of a gel containing a solvent, a liquid crystal spinning method of forming a fiber with the use of a liquid crystal solution or a melt, and the like), an approach of forming a piezoelectric polymer into a fiber by electrostatic spinning, or the like. It is to be noted that the cross-sectional shape of the piezoelectric fiber 10 is not to be considered limited to any circular shape.

The antibacterial yarn 1 constitutes a yarn (multifilament yarn) obtained by twisting a plurality of PLLA piezoelectric fibers 10 as described above. The antibacterial yarn 1 is a counterclockwise turning thread (hereinafter, referred to as an S thread) twisted by turning the piezoelectric fibers 10 counterclockwise. The stretching direction 900 for each piezoelectric fiber 10 coincides with the axial direction of each piezoelectric fiber 10. Therefore, the extending direction 900 for the piezoelectric fiber 10 is inclined to the left with respect to the axial direction of the antibacterial yarn 1. The angle depends on the number of twists.

When a tension is applied to the antibacterial yarn 1 of such an S thread, a negative electric charge is generated on the surface of the antibacterial yarn 1, and a positive electric charge is generated inside.

The antibacterial yarn 1 generates an electric field depending on the potential difference produced by the foregoing electric charge. This electric field also leaks into the nearby space, thereby forming a coupled electric field with other parts. In addition, when the antibacterial yarn 1 is brought close to an object with a predetermined close potential, for example, a predetermined potential (including a ground potential), such as a human body, the electric potential generated in the antibacterial yarn 1 generates an electric field between the antibacterial yarn 1 and the object.

Conventionally, it has been known that the proliferation of bacteria and fungi can be inhibited by an electric field (see, for example, Tetsuaki Doito, Hiroshi Koryo, Hideaki Matsuoka, Junichi Koizumi, Kodansha: Microbial Control-Science and Engineering, see for example, Koichi Takagi, Application of High Voltage Plasma Technology to Agriculture Food Field, and see J. HTSJ, Vol. 51, No. 216). In addition, due to the electric potential that generates the electric field, electric current may flow through a current pathway formed by moisture or the like, or a circuit formed by a local micro discharge phenomenon or the like. This electric current is considered to weaken bacteria and inhibit the proliferation of bacteria. It is to be noted that the bacteria referred to in the present embodiment include bacteria, fungi, and microorganisms such as mites and fleas.

Therefore, the antibacterial yarn 1 directly exerts an antibacterial effect with an electric field that is formed in the vicinity of the antibacterial yarn 1, or with an electric field that is generated when the antibacterial yarn 1 is brought close to an object with a predetermined electric potential, such as a human body. Alternatively, the antibacterial yarn 1 passes an electric current when the antibacterial yarn 1 is brought close to another nearby fiber or an object with a predetermined electric potential, such as a human body, via moisture such as sweat. Even with this electric current, an antibacterial effect may be directly exerted in some cases. Alternatively, an antibacterial effect may be indirectly exerted in some cases with active oxygen species in which oxygen included in moisture is altered by the action of an electric current or a voltage, radical species produced by interaction with or catalytic action of an additive material further included in the fiber, or another antibacterial chemical species (amine derivatives, and the like). Alternatively, oxygen radicals may be produced in bacterial cells by a stress environment due to the presence of an electric field or an electric current, thereby the antibacterial yarn 1 may indirectly exert an antibacterial effect in some cases. The generation of a superoxide anion radical (active oxygen) or a hydroxyl radical is considered as the radical. It is to be noted that the term "antimicrobial" in the present embodiment refers to a concept that includes both an effect of suppressing the generation of bacteria and an effect of killing bacteria.

The antibacterial yarn 1 as described above can be applied to various types of clothing or products such as medical members. For example, the antibacterial yarn 1 can be used for underwear (especially socks), towels, insoles of shoes, boots, and the like, general sportswear, hats, beddings (including futon, mattresses, sheets, pillows, pillowcases, and the like), toothbrushes, dental floss, various types of filters (filters of water purifiers, air conditioners, air purifiers, or the like), stuffed animals, pet-related items (pet mats, pet clothes, inners for pet clothes), various types of mats (for feet, hands, toilet seat, and the like), curtains, kitchen utensils (sponges, dishcloths, or the like), seats (seats of cars, trains, or airplanes), cushioning materials for motorcycle helmets and exterior materials therefor, sofas, bandages, gauze, masks, sutures, clothes for doctors and patients, supporters, sanitary goods, sporting goods (wear and inner gloves, gauntlets for use in martial arts, or the like), packaging materials, or the like.

Of the clothing, in particular, the socks (or supporters) are inevitably expanded and contracted along joints by movements such as walking, and the antibacterial yarn 1 thus generates electric charges with high frequency. In addition, although socks absorb moisture such as sweat and serve as hotbeds for proliferation of bacteria, the antibacterial yarn 1 can inhibit the proliferation of bacteria, and thus has a remarkable effect as a countermeasure against bacteria for deodorization.

In addition, the antibacterial yarn can also be used as a method for inhibiting bacteria on the body surfaces of animals excluding human beings, and may be disposed so that a cloth including a piezoelectric body is opposed to at least a part of the skin of an animal, for inhibiting the proliferation of bacteria on the body surface of the animal opposed to the cloth by the electric charge generated when an external force is applied to the piezoelectric body. Thus, a simple method makes it possible to inhibit the proliferation of bacteria on the body surface of an animal, which is safer than the use of a medicine or the like, and to treat ringworm fungus on the body surface of the animal.

Further, WO 2015/159832 discloses a transducer that has a plurality of piezoelectric yarns and conductive yarns made into a knitted fabric or a woven fabric and senses that displacement is applied to the knitted fabric or the woven fabric. In this case, all of the conductive yarns are connected to a detection circuit, and there is necessarily a pair of conductive yarns for one piezoelectric yarn. According to WO 2015/159832, when electric charges are generated in the piezoelectric yarns, electrons move through the conductive yarns, and immediately neutralize the electric charges generated in the piezoelectric yarns. According to WO 2015/159832, the detection circuit captures the electric current through the movement of the electrons, and outputs the current as a signal. Therefore, in this case, because the generated electric potential is canceled immediately, a strong electric field is not formed between the piezoelectric yarn and the conductive yarn and between the piezoelectric yarn and the piezoelectric yarn, and any antibacterial effect is not exerted.

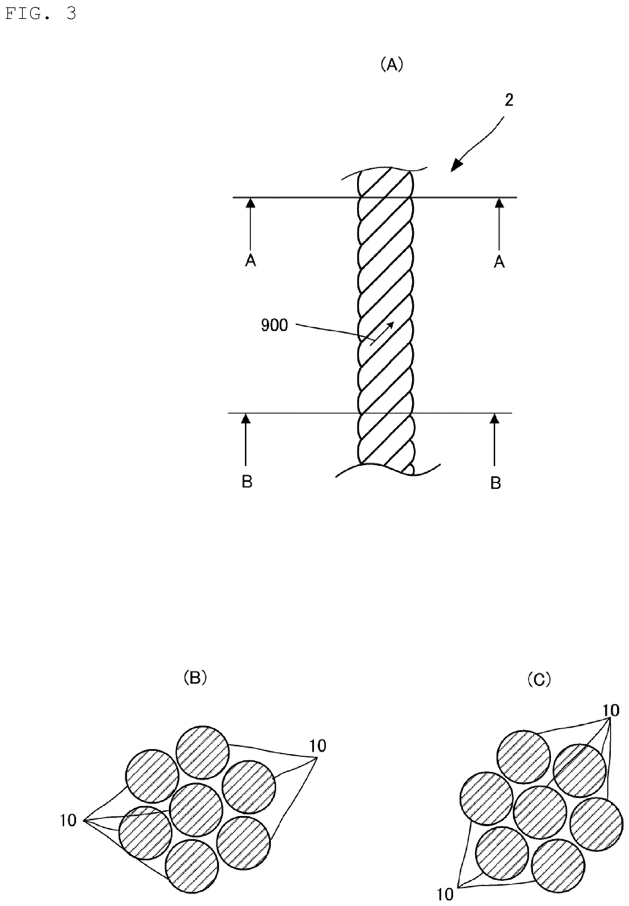

Next, FIG. 3(A) is a partially exploded view illustrating the configuration of the antibacterial yarn 2 constituting a clockwise-turning yarn (hereinafter, referred to as a Z thread) twisted by turning the piezoelectric fibers 10 clockwise. FIG. 3(B) is a cross-sectional view taken along the line A-A in FIG. 3 (A). FIG. 3(C) is a cross-sectional view taken along the line B-B in FIG. 3 (A).

The antibacterial yarn 2 is a Z thread, and the extending direction 900 of the piezoelectric fiber 10 is thus inclined to the right with respect to the axial direction of the antibacterial yarn 2. The angle depends on the number of yarn twists.

When a tension is applied to the antibacterial yarn 2 of such an Z thread, a positive electric charge is generated on the surface of the antibacterial yarn 2, and a negative electric charge is generated inside.

The antibacterial yarn 2 also generates an electric field depending on the potential difference produced by the foregoing electric charge. This electric field also leaks into the nearby space, thereby forming a coupled electric field with other parts. In addition, when the potential generated in the antibacterial yarn 2 is close to an object having a predetermined potential close to it, for example, a predetermined potential (including a ground potential) such as a human body, an electric field is generated between the antibacterial yarn 2 and the object.

Furthermore, an antibacterial yarn in which the antibacterial yarn 1 which is an S thread and the antibacterial yarn 2 which is a Z thread are brought close to each other can generate an electric field between the antibacterial yarn 1 and the antibacterial fiber 2.

The polarities of electric charges generated are different from each other between the antibacterial yarn 1 and the antibacterial yarn 2. The potential difference at each point is defined by an electric field coupling circuit formed by complicated entanglement of the fibers or a circuit formed by a current path accidentally formed in the thread with moisture or the like.

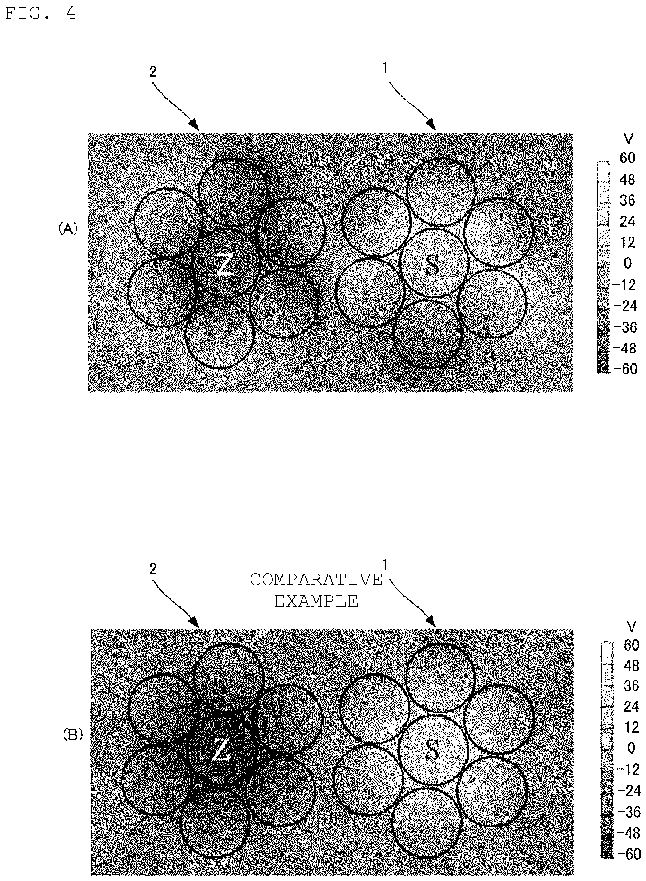

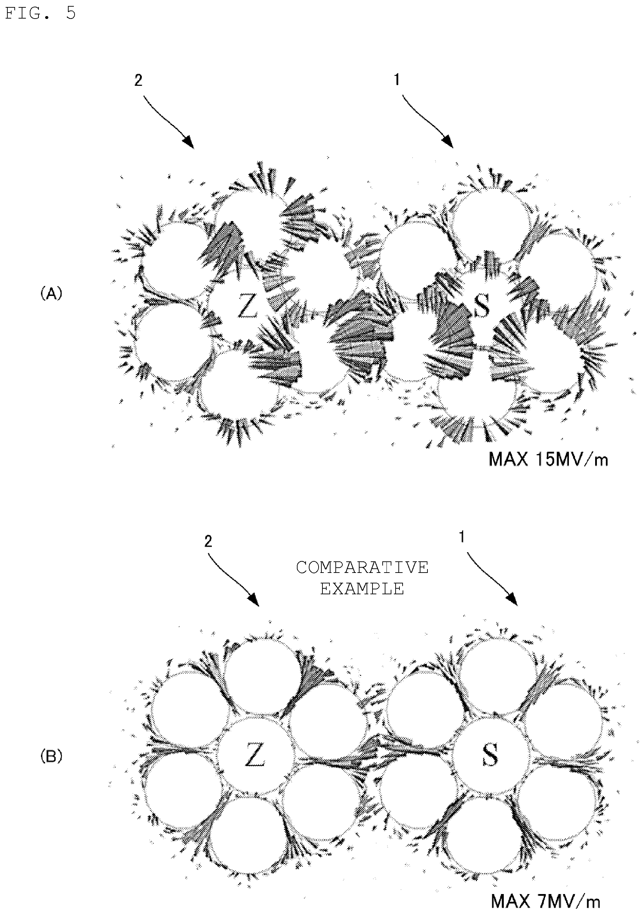

FIG. 4(A) is a diagram showing potentials in the antibacterial yarn 1 and the antibacterial yarn 2. In addition, FIG. 5(A) is a diagram showing an electric field. FIG. 4(B) is a diagram showing, as a comparative example, potentials in the case where the generated potentials for the plurality of piezoelectric fibers 10 in the antibacterial yarn 1 and the antibacterial yarn 2 are rotationally symmetric with respect to the center of the twisted yarns (comparative example). FIG. 5(B) is a diagram showing, as a comparative example, an electric field in the case where the generated potentials for the plurality of piezoelectric fibers 10 in the antibacterial yarn 1 and the antibacterial yarn 2 are rotationally symmetric with respect to the center of the twisted yarns (comparative example). It is to be noted that according to the present embodiment, as an example, the antibacterial yarn of the seven piezoelectric fibers 10 twisted is shown, but the number of twists is set appropriately in view of use applications and the like in practice.

When the antibacterial yarn 1 (S thread) and the antibacterial yarn 2 (Z thread) are formed from PLLA, the antibacterial yarn 1 alone has a negative electric potential on the surface and a positive electric potential inside when a tension is applied. The antibacterial yarn 2 alone has, when a tension is applied, a positive electric potential on the surface and a negative electric potential inside when a tension is applied.

When the antibacterial yarn 1 and the antibacterial yarn 2 are brought close to each other, the nearby parts (surfaces) tend to have the same electric potential. In this case, the nearby parts of the antibacterial yarn 1 and the antibacterial yarn 2 reach 0 V, and the positive electric potential inside the antibacterial yarn 1 is further increased so as to keep the original potential difference. Likewise, the negative electric potential inside the antibacterial yarn 2 is further decreased.

In a cross section of the antibacterial yarn 1, an electric field directed mainly outward from the center is formed, and in a cross section of the antibacterial yarn 2, an electric field directed mainly inward from the center is formed. When the antibacterial yarn 1 and the antibacterial yarn 2 are brought close to each other, the foregoing electric fields leak into the air and then combine to form an electric field circuit between the antibacterial yarn 1 and the antibacterial yarn 2.

In this regard, if the spaces between the plurality of piezoelectric fibers 10 in the antibacterial yarn 1 and the antibacterial yarn 2 are uniform as in the comparative example shown in FIG. 4(B), there is no bias in the electric potential distribution, with the highest electric potential at the center of the antibacterial yarn 1 and the lowest electric potential at the center of the antibacterial yarn 2. Therefore, as in the comparative example shown in FIG. 5(B), the electric field formed between the antibacterial yarn 1 and the antibacterial yarn 2 reach a maximum in the space where the antibacterial yarn 1 and the antibacterial yarn 2 are close to each other, and the electric fields formed in the other spaces are not so large.

In contrast, in the antibacterial yarn according to the present embodiment, the charge generation patterns of the plurality of piezoelectric fibers 10 are not rotationally symmetrical with respect to the center of the twisted yarns. For example, as shown in FIGS. 1(B) and 1(C), the antibacterial yarn 1 differs in the arrangement aspect of the plurality of piezoelectric fibers 10 between in the case of viewing a certain cross section and in the case of viewing another certain cross section. In addition, as shown in FIGS. 3(B) and 3(C), the antibacterial yarn 2 also differs in the arrangement aspect of the plurality of piezoelectric fibers 10 between in the case of viewing a certain cross section and in the case of viewing another certain cross section. Furthermore, in any of these cross sections, the direction of the shear stress applied to the piezoelectric fiber 10 is not rotationally symmetric with respect to the center of the yarns, and the shear stress also varies in strength. Such non-rotational symmetry of the generated electric potentials due to the piezoelectric effect of the piezoelectric fiber 10 is caused by positively adding the following various causes. The non-rotational symmetry is caused when the piezoelectric fibers 10 differ in diameter, when the piezoelectric fibers 10 differ in shape, or when the distances between the piezoelectric fibers 10 are different from each other. Alternatively, the non-rotational symmetry is caused when the foregoing conditions are developed in a compositive manner, etc.

With this configuration, as shown in FIG. 4(A), the electric potential distribution of the antibacterial yarn according to the embodiment is biased, thereby destroying the symmetry, and thus, a strong electric field (a stronger electric field than according to the comparative example) will be formed locally as shown in FIG. 5(A). For example, in the example shown in FIG. 5(B), the electric field is at most 7 MV/m, but in the antibacterial yarn shown in FIG. 5(A), an electric field up to 15 MV/m is generated. Therefore, the antibacterial yarn according to the present embodiment can generate a stronger electric field than in the case of a plurality of piezoelectric fibers 10 uniformly arranged.

Further, an example of the case where the generated potential due to the piezoelectric effect of the plurality of piezoelectric fibers 10 is not rotationally symmetrical with respect to the center of the yarns will be described more specifically as follows.

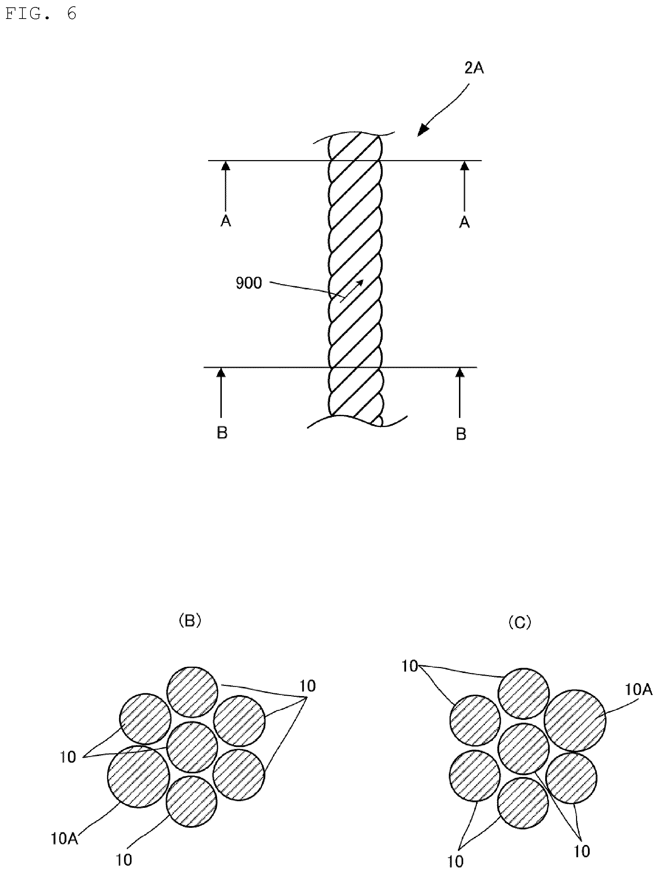

FIG. 6(A) is a partially exploded view illustrating the configuration of an antibacterial yarn according to Modification Example 1, and FIG. 6(B) is a cross-sectional view taken along the line A-A in FIG. 6(A). FIG. 6(C) is a cross-sectional view taken along the line B-B of FIG. 6(A). The same constituents as those of the antibacterial yarn 2 shown in FIG. 3 are denoted by the same reference numerals, and descriptions thereof will be omitted.

The antibacterial yarn 2A includes a piezoelectric fiber 10A that differs in thickness from the piezoelectric fiber 10. As described above, also in the case of partially including antibacterial fibers that differ in thickness, the spaces between the plurality of piezoelectric fibers 10 are not uniform. In addition, the tension applied to each piezoelectric fiber 10 is also not constant, and the direction of shear stress is also not uniform. Therefore, the electric potential distribution is biased, thereby destroying the symmetry, and a strong electric field (a stronger electric field than according to the comparative example) will be formed locally.

In addition, there is no need for the diameter of the antibacterial yarn 2A to be constant in the longitudinal direction, and the antibacterial yarn 2A may be partially larger or smaller in diameter. In FIG. 6(B) and FIG. 6(C), there is one piezoelectric fiber that differs in thickness, but there may be multiple piezoelectric fibers that differ in thickness. In addition, for example, the material referred to as the piezoelectric fiber 10A may be different from the material of the other piezoelectric fibers 10. In this case, the piezoelectric fiber 10A is different in piezoelectric tensor from the other piezoelectric fibers 10. In this case, the piezoelectric fiber 10A also includes a case where no piezoelectricity is exhibited. Also in this case, the electric potential distribution is biased, thereby destroying the symmetry, and a strong electric field (a stronger electric field than according to the comparative example) will be formed locally. As just described, the fibers may be equal in thickness as long as an element that destroys an electrical symmetry is included.

It is to be noted that even in the case of partially including piezoelectric fibers that differ in cross-sectional shape (for example, a circular piezoelectric fiber and a polygonal piezoelectric fiber), the spaces between the plurality of piezoelectric fibers 10 are not uniform.

It is to be noted that, the antibacterial yarn 2A of the Z thread is shown in FIGS. 6(A), 6(B), and 6(C), but as a matter of course, also when an antibacterial yarn of an S thread partially has piezoelectric fibers that differ in thickness or partially has fibers that differ in cross-sectional shape, the spaces between the plurality of piezoelectric fibers 10 are not uniform, the electric potential distribution is thus biased, thereby destroying the symmetry, and a strong electric field (a stronger electric field than according to the comparative example) will be formed locally.

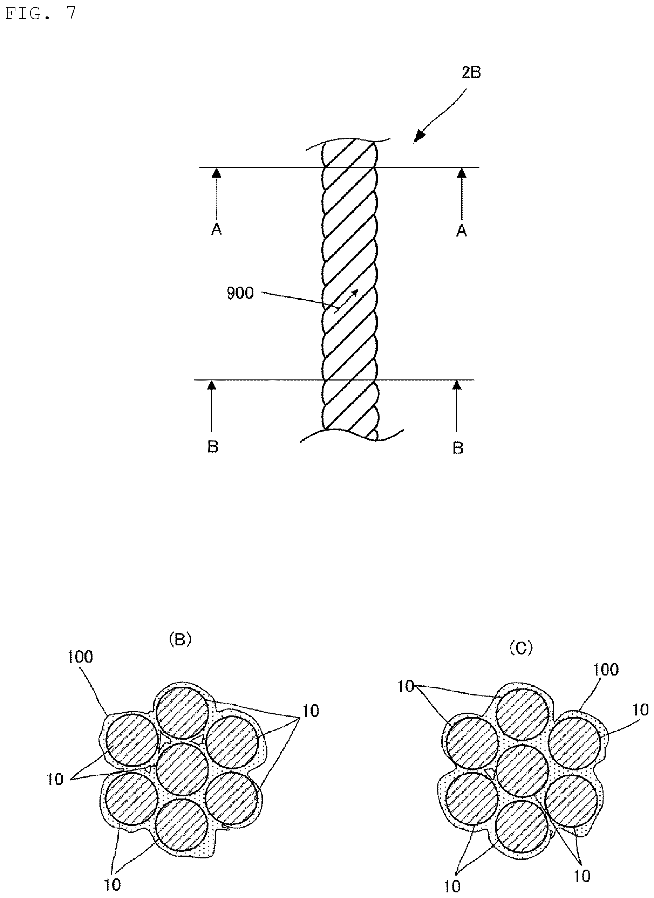

FIG. 7(A) is a partially exploded view illustrating the configuration of an antibacterial yarn 2B according to Modification Example 2, and FIG. 7(B) is a cross-sectional view taken along the line A-A in FIG. 7(A). FIG. 7(C) is a cross-sectional view taken along the line B-B of FIG. 7(A). The same constituents as those of the antibacterial yarn 2 shown in FIG. 3 are denoted by the same reference numerals, and descriptions thereof will be omitted.

The antibacterial yarn 2B further includes a dielectric 100 between a plurality of piezoelectric fibers 10. It is to be noted that the dielectric 100 covers the piezoelectric fibers 10 in the example of FIGS. 7(B) and 7(C), but it is not indispensable to cover all of the piezoelectric fibers 10. However, covering the piezoelectric fibers 10 with the use of a flame retardant as the dielectric 100 makes it possible to provide a flame-retardant antibacterial yarn for use in highly public seats (or seat covers), such as a car seat, an electric train seat, a bus seat, a theater seat, and a hospital (waiting room) seat.

For the flame retardant, for example, a bromine compound (for example, pentabromodiphenyl ether, octabromodiphenyl ether, decabromodiphenyl ether, tetrabromobisphenol A, hexabromocyclododecane, hexabromobenzene) is applied to the piezoelectric fibers 10. Alternatively, a phosphorus compound (for example, an aromatic phosphate ester, a phosphate ester including a halogen), a chlorine compound (for example, chlorinated paraffin), or an antimony compound (for example, antimony pentoxide) is applied to the surfaces of the piezoelectric fibers 10. The flame retardant may have antimony trioxide, aluminum hydroxide, magnesium hydroxide, hydroxyapatite, melamine cyanurate, best boron, SOUFA, talc, or silica kneaded in a base material.

For the antibacterial yarn 2B, as shown in FIGS. 7(B) and 7(C), the dielectric 100 is disposed between the plurality of piezoelectric fibers 10 in a non-uniform fashion. The dielectric 100 is disposed in a non-uniform fashion, thereby, in the antibacterial yarn 2B, making the distances between the plurality of piezoelectric fibers 10 non-uniform, or biasing electrical characteristics even if the distances are uniform between the plurality of piezoelectric fibers 10. Therefore, also in this case, the spaces between the plurality of piezoelectric fibers 10 are not uniform, and a strong electric field (a stronger electric field than according to the comparative example) will be formed locally.

It is to be noted that the antibacterial yarn 2B of the Z thread is shown in FIGS. 7(A), 7(B), and 7(C), but as a matter of course, also when an antibacterial yarn of an S thread is provided with a dielectric 100 with the dielectric 100 disposed in a non-uniform fashion, the spaces between the plurality of piezoelectric fibers 10 are not uniform, and a strong electric field (a stronger electric field than according to the comparative example) will be formed locally.

Further, the antibacterial yarn according to the present embodiment has the following use applications other than countermeasures against bacteria.

### (1) Bioactive Piezoelectric Yarn

Many tissues constituting a living body have piezoelectricity. For example, collagen constituting a human body, which is a kind of protein, is included a lot in blood vessels, dermis, ligaments, tendons, bones, cartilages, and the like. Collagen is a piezoelectric body, and a tissue with collagen oriented may exhibit a great deal of piezoelectricity. Many reports have already been made on the piezoelectricity of bones (see, for example, Eiichi Fukada, Piezoelectricity of Biopolymer, Polymer Vol. 16 (1967) No. 9 p795-800, etc.). Therefore, when the antibacterial yarn 1 or the antibacterial yarn 2 generates an electric field, alternates the electric field or changes the strength of the electric field, the piezoelectric body of a living body vibrates due to the inverse piezoelectric effect. The alternated electric field or the change in the electric field strength, generated by the antibacterial yarn 1 or the antibacterial yarn 2, applies a minute vibration to a part of a living body, for example, a capillary blood vessel or dermis, thereby making it possible to encourage improvement in blood flow through the part. Thus, there is a possibility that the healing of skin diseases, wounds, and the like may be promoted. Therefore, the antibacterial yarn functions as a bioactive piezoelectric yarn.

Further, the transducer that has a plurality of piezoelectric yarns and conductive yarns made into a knitted fabric or a woven fabric and senses that displacement is applied to the knitted fabric or the woven fabric is disclosed in WO 2015/159832. In this case, all of the conductive yarns are connected to a detection circuit, and there is necessarily a pair of conductive yarns for one piezoelectric yarn. According to WO 2015/159832, when electric charges are generated in the piezoelectric yarns, electrons move through the conductive yarns, and immediately neutralize the electric charges generated. According to WO 2015/159832, the detection circuit captures the electric current through the movement of the electrons, and outputs the current as a signal. Therefore, in this case, because the generated electric potential is canceled immediately, a strong electric field is not formed between the piezoelectric yarn and the conductive yarn and between the piezoelectric yarn and the piezoelectric yarn, and any healing effect is not exerted.

### (2) Piezoelectric Yarn for Substance Adsorption

As described above, the antibacterial yarn 1 generates a negative electric charge when an external force is involved. The antibacterial yarn 2 generates a positive electric charge when an external force is involved. Therefore, the antibacterial yarn 1 has the property of adsorbing a substance with a positive electric charge (for example, particles such as pollens), and the antibacterial yarn 2 adsorbs a substance that has a negative electric charge (for example, harmful substances such as yellow sand). Therefore, a cloth including the antibacterial yarn 1 or the antibacterial yarn 2 can adsorb fine particles such as pollens or yellow sand, when the cloth is applied to a medical supply such as a mask.

As described above, the transducer that has a plurality of piezoelectric yarns and conductive yarns made into a knitted fabric or a woven fabric and senses that displacement is applied to the knitted fabric or the woven fabric is disclosed in WO 2015/159832. In this case, all of the conductive yarns are connected to a detection circuit, and there is necessarily a pair of conductive yarns for one piezoelectric yarn. According to WO 2015/159832, when electric charges are generated in the piezoelectric yarns, electrons move through the conductive yarns, and immediately neutralize the electric charges generated. According to WO 2015/159832, the detection circuit captures the electric current through the movement of the electrons, and outputs the current as a signal. Therefore, in this case, because the generated electric potential is canceled immediately, a strong electric field is not formed between the piezoelectric yarn and the conductive yarn and between the piezoelectric yarn and the piezoelectric yarn, and any adsorption effect is not exerted.

It is to be noted that in addition to the S thread that uses PLLA, a Z thread that uses PDLA is also conceivable as a fiber that generates a negative electric charge on the surface. In addition to the Z thread that uses PLLA, an S thread that uses PDLA is also conceivable as a fiber that generates a positive electric charge on the surface.

Finally, the descriptions of the present embodiment should be considered exemplary in all respects, but not be considered limiting. The scope of the present invention is specified by the claims, but not by the embodiments described above.

### Description Of Reference Symbols

1, 2, 2A, 2B: antibacterial yarn
10,10A: piezoelectric fiber
100: dielectric
900: stretching direction
910A: first diagonal line
910B: second diagonal line

## Claims

1. An antibacterial twisted yarn (1, 2) comprising a plurality of charge generation fibers (10) that generate an electric charge with external energy, wherein the charge generation fibers (10) comprise a piezoelectric polymer,
wherein the charge generation fibers (10) have one or more fiber properties such that an arrangement aspect between the plurality of charge generation fibers is not uniform, the one or more fiber properties including: a non-uniform fiber cross-sectional area, a non-uniform fiber cross-sectional shape, and non-uniform fiber diameters,
and wherein non-rotationally symmetric electric potentials arise with respect to the center of the twisted yarn due to the non-uniform arrangement aspect.

2. The antibacterial twisted yarn of claim 1, wherein a non-uniform fiber cross-sectional shape includes charge generation fibers with a circular cross-section and charge generation fibers with a polygonal cross-section.

3. The antibacterial twisted yarn according to claim 1,
further comprising a dielectric between the plurality of charge generation fibers, wherein the dielectric (100) is disposed in a non-uniform fashion.

4. The antibacterial twisted yarn according to claim 3, wherein the dielectric (100) coats the charge generation fibers.

5. The antibacterial twisted yarn according to claim 3 or 4, wherein the dielectric (100) comprises a flame retardant material.

6. A seat comprising the antibacterial twisted yarn according to any of claims 1 to 5.

7. A seat cover comprising the antibacterial twisted yarn according to any one of claims 1 to 5.

## Patentansprüche

1. Antibakterieller Zwirn (1, 2), der mehrere Ladungserzeugungsfasern (10) umfasst, die mit externer Energie eine elektrische Ladung erzeugen, wobei die Ladungserzeugungsfasern (10) ein piezoelektrisches Polymer umfassen,
wobei die Ladungserzeugungsfasern (10) eine oder mehrere Fasereigenschaften aufweisen, so dass ein Anordnungsaspekt zwischen den mehreren Ladungserzeugungsfasern nicht einheitlich ist, wobei die eine oder mehreren Fasereigenschaften Folgendes einschließen: eine ungleichmäßige Faserquerschnittsfläche, eine ungleichmäßige Faserquerschnittsform und ungleichmäßige Faserdurchmesser, und
wobei aufgrund des ungleichmäßigen Anordnungsaspekts nicht rotationssymmetrische elektrische Potentiale in Bezug auf die Mitte des Zwirns entstehen.

2. Antibakterieller Zwirn nach Anspruch 1, wobei eine ungleichmäßige Faserquerschnittsform Ladungserzeugungsfasern mit einem kreisförmigen Querschnitt und Ladungserzeugungsfasern mit einem polygonalen Querschnitt einschließt.

3. Antibakterieller Zwirn nach Anspruch 1,
der ferner ein Dielektrikum zwischen den mehreren Ladungserzeugungsfasern umfasst, wobei das Dielektrikum (100) ungleichmäßig angeordnet ist.

4. Antibakterieller Zwirn nach Anspruch 3,
wobei das Dielektrikum (100) die Ladungserzeugungsfasern umhüllt.

5. Antibakterieller Zwirn nach Anspruch 3 oder 4, wobei das Dielektrikum (100) ein flammhemmendes Material umfasst.

6. Sitz, der den antibakteriellen Zwirn nach einem der Ansprüche 1 bis 5 umfasst.

7. Sitzbezug, der den antibakteriellen Zwirn nach einem der Ansprüche 1 bis 5 umfasst.

## Revendications

1. Fil torsadé antibactérien (1, 2) comprenant une pluralité de fibres génératrices de charge (10) qui génèrent une charge électrique avec de l'énergie externe, dans lequel les fibres de génération de charge (10) comprennent un polymère piézoélectrique,
dans lequel les fibres de génération de charge (10) ont une ou plusieurs propriétés de fibre telles qu'un aspect d'agencement entre la pluralité de fibres de génération de charge n'est pas uniforme, les une ou plusieurs propriétés de fibre comportant : une section transversale de fibre non uniforme, une forme de section transversale de fibre non uniforme et des diamètres de fibre non uniformes,
et dans lequel des potentiels électriques non symétriques en rotation se produisent par rapport au centre du fil torsadé en raison de l'aspect d'agencement non uniforme.

2. Fil torsadé antibactérien selon la revendication 1, dans lequel une forme de section transversale de fibre non uniforme comprend des fibres génératrices de charge à section transversale circulaire et des fibres génératrices de charge à section transversale polygonale.

3. Fil torsadé antibactérien selon la revendication 1, comprenant en outre un diélectrique entre la pluralité de fibres génératrices de charge, dans lequel le diélectrique (100) est disposé de manière non uniforme.

4. Fil torsadé antibactérien selon la revendication 3, dans lequel le diélectrique (100) recouvre les fibres de génération de charge.

5. Fil torsadé antibactérien selon la revendication 3 ou 4, dans lequel le diélectrique (100) comprend un matériau ignifuge.

6. Siège comprenant le fil torsadé antibactérien selon l'une quelconque des revendications 1 à 5.

7. Housse de siège comprenant le fil torsadé antibactérien selon l'une quelconque des revendications 1 à 5.
